Europäisches Patentamt

European Patent Office

Office européen des brevets

(11) Publication number: **0 427 401 A1**

# EUROPEAN PATENT APPLICATION

(21) Application number: 90311133.4

(22) Date of filing: **11.10.90**

(51) Int. Cl.⁵: **C07C 227/38, C07C 229/16**

(30) Priority: **11.10.89 US 419919**

(43) Date of publication of application:
**15.05.91 Bulletin 91/20**

(84) Designated Contracting States:
**BE CH DE DK ES FR GB IT LI NL SE**

(71) Applicant: W.R. Grace & Co.-Conn.
**1114 Avenue of the Americas**
**New York New York 10036(US)**

(72) Inventor: Thunberg, Jon C.
**RFD No. 3 Mont Vernon Street**
**Milford, New Hampshire 03055(US)**
Inventor: Tiffany, Daniel R.
**410 Cole Street**
**San Franciso, California 94117(US)**

(74) Representative: Bentham, Stephen et al
**J.A. Kemp & Co. 14 South Square Gray's Inn**
**London, WC1R 5LX(GB)**

(54) Conversion of iminodiacetic acid crystal liquors to concentrated solutions of monoalkalimetal or monoammonium iminodiacetate.

(57) Conversion of iminodiacetic acid crystal liquors to solutions of monoalkalimetal or monoammonium iminodiacetate is disclosed. The process involves adjusting the pH of the IDA liquor to about 6-7 with an alkalimetal or ammonium base, and cooling the solution. Where the starting solution comprises substantial amounts of sodium sulfate, sodium sulfate decahydrate can be separated by cooling the solution to the saturation temperature of sodium sulfate decahydrate.

EP 0 427 401 A1

# CONVERSION OF IMINODIACETIC ACID CRYSTAL LIQUORS TO CONCENTRATED SOLUTIONS OF MON-OALKALIMETAL OR MONOAMMONIUM IMINODIACETATE

This invention relates to the conversion of iminodiacetic acid liquors, such as liquor generated in the process of producing iminodiacetic acid, to concentrated solutions of monoalkalimetal or monoammonium iminodiacetate.

Typical prior art processes for the recovery of iminodiacetic acid from sodium sulfate solutions are disclosed in U.S. Patents 3,808,269 and 4,299,978.

U.S. Patent No. 3,808,269, the disclosure of which is herein incorporated by reference, discloses a process of recovering iminodiacetic acid (IDA) from a starting aqueous solution of sodium sulfate and the amino acid having a temperature above about 33°C and containing at least 5% amino acid. The process comprises adjusting the pH of the starting solution to 1.5-3 to form an IDA precipitate and a first mother liquor; separating the IDA precipitate from the first mother liquor; and recovering the separated IDA. Sodium sulfate can then be precipitated from the first mother liquor by concentrating that liquor and adjusting the temperature so as to prevent precipitation of IDA.

U.S. Patent No. 4,299,978, the disclosure of which is herein incorporated by reference, discloses a process for separating an "iminodiacetic acid component" from an aqueous glycine solution including IDA. The process comprises adding sulfuric acid in the presence of a sodium salt to the aqueous solution so as to lower the pH to 1.5 or less, whereby an "iminodiacetic acid component" is crystallized from the solution, and separating the precipitated IDA component. Glycine can thus be efficiently recovered with minimal levels of IDA. Glauber's salt is not generated.

The foregoing references use processes where the precipitation of sodium sulfate with the amino acid is avoided. These processes generate waste liquor streams in which the concentration of IDA is too low to serve any known purpose. Streams such as this have heretofore been discarded.

The problems of the prior art have been overcome by the present invention which provides a process for conversion of iminodiacetic acid liquors to solutions of the monoalkalimetal or monoammonium salt of iminodiacetic acid. The resulting solutions can serve as intermediates in organic synthesis where the impurities therein are not detrimental to the synthesis.

It is therefore an object of the present invention to provide a process to minimize generation of waste from the production of IDA.

It is a further object of the present invention to provide a process for the recovery of value from the waste generated from the production of IDA.

It is a still further object of the present invention to provide a process which reduces disposal costs in the production of IDA.

In accordance with the present invention, these and other objects of the invention are accomplished by providing a process for converting IDA liquors to concentrated solutions of the monoalkalimetal or monoammonium salt of IDA, which comprises adjusting the pH of such solutions to about 6-7 with an alkalimetal or ammonium base, and cooling the neutralized liquor.

The process of preparing IDA from the corresponding nitrile can be accomplished according to the following sequence of reactions:

$$HN(CH_2CN)_2 + 2H_2O + 2NaOH \longrightarrow HN(CH_2COONa)_2 + 2NH_3$$
$$HN(CH_2COONa)_2 + H_2SO_4 \longrightarrow HN(CH_2COOH)_2 + Na_2SO_4$$

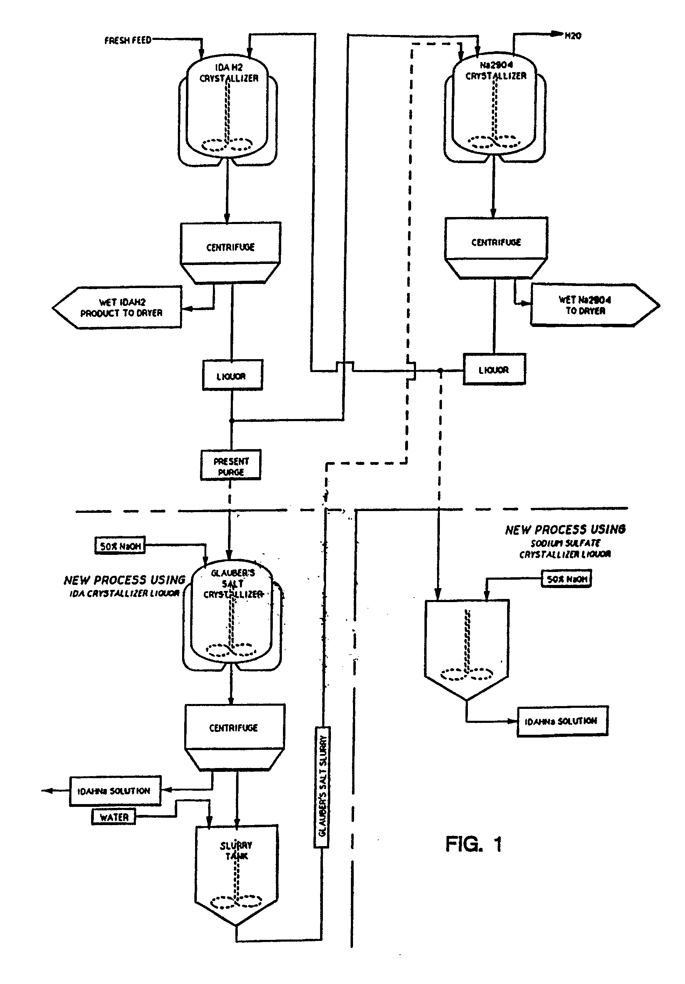

Turning now to Figure 1, the upper portion thereof illustrates a process for recovering iminodiacetic acid from the sodium sulfate solutions resulting from the above-depicted process. Feed is introduced into a crystallizer where IDA is crystallized and subsequently centrifuged. The resulting mother liquor is fed to a second crystallizer where sodium sulfate is crystallized and subsequently centrifuged. The mother liquor resulting from the sodium sulfate separation is recycled to the IDA crystallizer.

In a first embodiment of the present invention, solution comprising IDA and sodium sulfate, such as a portion of the mother liquor resulting from the aforementioned IDA separation, is fed to a crystallizer. This solution typically has a temperature of about 40°C, at which it is saturated with IDA (IDA content of about 6.5% by weight). The solution is neutralized to a pH at which the monosalts of IDA are formed. The temperature is adjusted to about the saturation temperature of sodium sulfate decahydrate to precipitate sodium sulfate decahydrate, and then can be cooled to a desired temperature, such as 5°C, where the monosalts of IDA are still soluble. The neutralization preferably is carried out to a pH of about 6-7. No IDA precipitates with, but a small amount is occluded on, the precipitated sodium sulfate decahydrate. Suitable neutralizing agents are appropriately chosen to form soluble IDA salts, and include alkalimetal hydroxides and ammonium hydroxide. Sodium and potassium hydroxides are preferred alkalimetal hydroxides, with sodium hydroxide being especially preferred.

Sodium sulfate decahydrate seed crystals can be added to the solution to facilitate the crystallization of sodium sulfate decahydrate. This crystallization removes most of the sodium sulfate and produces a liquor with low sulfate content. The simultaneous removal of ten moles of water per mole of removed sodium sulfate also contributes to the concentration of the mother liquor. The precipitated sodium sulfate decahydrate can be separated, such as by centrifugation or decantation. The separated wet cake of sodium sulfate decahydrate can be recycled to the sodium sulfate crystallizer in the IDA separation process by forming a pumpable slurry.

In a second embodiment of the present invention, the feed stream to the crystallizer is at least a portion of the mother liquor after sodium sulfate separation. This liquor typically has a temperature of about $80°C$, at which temperature it is saturated with IDA (IDA concentration of about 17% by weight). Since most of the sulfate has been removed, expensive solid handling equipment to handle sodium sulfate decahydrate is not necessary. The solution is neutralized to a pH at which the monoalkalimetal or monoammonium salts of IDA are formed, preferably to a pH of about 6-7. The temperature can then be cooled to a desired level, such as $20°C$, where the salt remains soluble. Some sodium sulfate may precipitate, which can be filtered. If such precipitation is objectional, the solution can be diluted with water. Suitable neutralizing agents are appropriately chosen to form soluble IDA salts and include alkalimetal and ammonium hydroxides. Sodium and potassium hydroxides are preferred, with sodium hydroxide being especially preferred.

The instant invention will be better understood by referring to the following specific but non-limiting examples. It is understood that said invention is not limited by these procedures which are offered merely as illustrations; it is also understood that modifications can be made without departing from the spirit and scope of the invention.

All the percentages quoted in the Examples which follow are percentages by weight.

## EXAMPLE 1

1500 g of liquor having a composition comprising about 6.5% IDA acid and about 22% $Na_2SO_4$ was charged to a 1 L jacketed crystallizer, heated to $40°C$, and neutralized to pH 6.4 with 82.6 g of 50% NaOH. The solution was cooled to $28°C$ and seeded with 10.0 g of $Na_2SO_4$ 10 $H_2O$ (Glauber's Salt, or G.S.) An exotherm of $0.6°C$ occurred, indicating that crystallization of G.S. had been initiated (i.e. saturation had been reached). The mixture was linearly cooled to $5°C$ over 4 hr, held at $5°C$ for 30 min, and centrifuged. The cake was not washed. The slurry was extremely thick and had a translucent appearance which actually made it quite difficult to see any crystals in the stirring slurry. Table I shows the mass analytical data.

## EXAMPLE 2

The purpose of this experiment was to demonstrate the recycle of mother liquor to control the slurry density. 900 g of liquor plus 600 g of $5°C$ mother liquor from the previous experiment was neutralized with 50.3 g of 50% NaOH to pH 6.4 and crystallized in exactly the same way as described above. The solution had to be seeded twice to attain saturation; once at $26°C$ and again at $24°C$. The $5°C$ slurry contained 16.8% IDAHNa, or 14.4% as $IDAH_2$, and 3.2% $Na_2SO_4$. Table I shows the mass and analytical data.

## EXAMPLE 3

### Direct Neutralization of $Na_2SO_4$ Liquor

1205 g of liquor having a composition comprising about 17% IDA acid and about 16% $Na_2SO_4$ was reheated to $80°C$ and neutralized to pH 6.6 with 173.8 g of 50% NaOH. A very fine precipitate was present which did not fully dissolve by the addition of 246 g of water. When cooled to $27°C$ no crystals were present, but the fine precipitate remained. Crystallization was not initiated by seeding with G.S. indicating that this would be a stable solution. 63 g of sample was removed and the solution was concentrated to 1325 g (equivalent to the original concentration with compensation for the sample removed). This mixture was cooled to approximately $20°C$, readjusted to 1325 g, and filtered to remove the precipitate. A sample of liquor was cooled to $5°C$ and seeded with G.S. - no crystallization occurred, thus demonstrating that this would be a stable solution. This solution contained 22.5% IDAHNa, or 19.3% $IDAH_2$, and 11.4% $Na_2SO_4$. Table I shows the mass and analytical data.

## EXAMPLE 4

500 g of liquor obtained from the sodium sulfate centrifuge liquor tank in the IDA recovery process illustrated in Fig. 1, and having a composition comprising about 12.6% $IDAH_2$, was neutralized with 51.3 g of 50% NaOH with the addition of water as needed to replace that lost by evaoporation. The cooled solution contained a small amount of solid which was removed by filtration. Upon drying, this solid melted, indicating that it was $Na_2SO_4.10H_2O$, or Glauber's Salt.

Table 2 shows the mass and analytical data.

## EXAMPLE 5

500 g of hot liquor obtained from the sodium sulfate centrifuge liquor tank in the IDA recovery process illustrated in Fig. 1, and having a composition comprising about 12.6% $IDAH_2$, was neutralized to pH 6.9 with 86.0 g of 45% KOH. This solution was cooled and filtered in the same way as in the above example. This solid did not melt upon drying, indicating what it was probably $K_2SO_4$.

Table 2 shows the mass and analytical data.

## EXAMPLE 6

500 g of hot liquor obtained from the sodium sulfate centrifuge liquor tank in the IDA recovery process illustrated in Fig. 1, and having a composition comprising about 12.6% $IDAH_2$, was neutralized to pH 6.9 with 40.3 g of about 28% $NH_4OH$. This solution did not developed crystals upon cooling.

Table 2 shows the mass and analytical data.

| | Example 1 Liquor (From IDA) | Example 2 Liquor (From IDA) | Example 3* Liquor (From Na2SO4) |
|---|---|---|---|
| **CHARGES:** | | | |
| Liquor | 1500 | 900 | 1150 |
| Recycled Liquor | 0 | 600 | 0 |
| 50% NaOH | 82.6 | 50.3 | 165.8 |
| G.S. Seed | 10 | 20 | 0 |
| Total | 1593 | 1570 | 1316 |
| **RECOVERED:** | | | |
| Wet Cake | 782.2 | 491.5 | 47.3 |
| New Liquor | 767.2 | 1047 | 1263.5 |
| Total | 1549 | 1539 | 1311 |
| Air Dried Cake | 344.9 | 216 | 41.1 |
| **ANALYSES OF DRY SOLIDS:** | | | |
| $Na_2SO_4$ | 99.60% | 99.20% | 93.70% |
| IDAH2 Expressed as IDAHNa | 1.51% | 1.51% | 2.52% |
| NTAH3 | 0.51% | 0.54% | 3.80% |
| **ANALYSES OF LIQUORS:** | | | |
| IDAH2 | 14.40% | 14.40% | 19.30% |
| $IDA_2$ Expressed as IDAHNa | 16.80% | 16.80% | 22.50% |
| NTAH3 | 5.50% | 5.50% | 6.80% |
| $Na_2SO_4$ | 3.20% | 3.20% | 11.40% |
| TOTAL SOLIDS | 31.67% | 31.67% | 47.63% |
| (IDAHNa + NTAH3 + Na2SO4) | 80.52% | 80.52% | 85.45% |
| SLURRY DENSITY | 49% | 31% | |

* Compensation for removal of sample is included in the data for this example.

| EXAMPLE NO. | 4 50% NaOH | 5 45% KOH | 6 28% NH4OH |
|---|---|---|---|
| Base | | | |
| **Grams Charged:** | | | |
| IDA Liquor | 500.0 | 500.0 | 500.0 |
| Base | 51.3 | 86.0 | 40.3 |
| Total | 551.3 | 586.0 | 540.3 |
| **Grams Recovered:** | | | |
| Wet Solid | 34.6 | 47.7 | 0.0 |
| Dry Solid | 17.8 | 41.2 | 0.0 |
| Solution | 505.8 | 536.0 | 540.3 |

| Analysis of Feedstock: | |
|---|---|
| IDAH2 | 12.60% |
| NTAH3 | 7.82% |
| Na$_2$SO4 | 19.60% |
| Total Solids | 48.78% |

| Analysis of Dried Solid: | | | |
|---|---|---|---|
| IDAH2 | 3.62% | 2.92% | 0.00% |
| NTAH3 | 2.23% | 2.22% | 0.00% |
| Na$_2$SO4 | 87.30% | 74.20% | 0.00% |

| Analysis of Solution: | | | |
|---|---|---|---|
| IDAH2 | 11.74% | 11.26% | 11.31% |
| NTAH3 | 5.58% | 5.42% | 5.59% |
| Na$_2$SO4 | 14.70% | 11.10% | 17.80% |
| Total Solids | 43.36% | 39.71% | 40.53% |

| % of IDA in Feedstock Recovered in Solution Produced | 94.3% | 95.8% | 97.0% |
|---|---|---|---|

## Claims

1. A process for converting an aqueous solution comprising iminodiacetic acid to an aqueous solution comprising iminomonoacetate, comprising

a. adjusting the pH of an aqueous solution comprising iminodiacetic acid to a pH of from 6 to 7 with an alkali metal hydroxide or ammonium hydroxide; and

b. cooling the solution.

2. A process according to claim 1 wherein said base is sodium hydroxide.

3. A process according to claim 1 wherein said base is potassium hydroxide.

4. A process for preparing an aqueous solution comprising:

a. adjusting the pH of an aqueous solution comprising iminodiacetic acid and sodium sulfate to a pH of from 6 to 7 with an alkali metal hydroxide or ammonium hydroxide;

b. cooling the resulting neutralized solution to a temperature effective for precipitating sodium sulfate decahydrate; and

c. separating said precipitated sodium sulfate decahydrate from the resulting mother liquor.

5. A process according to claim 4 wherein sodium sulfate decahydrate seed crystals are added at about the saturation temperature of sodium sulfate decahydrate.

6. A process according to claim 4 or 5 further comprising cooling the mother liquor.

7. A process according to any one of claims 4 to 6 wherein said base is sodium hydroxide.
8. A process according to any one of claims 4 to 6 wherein said base is potassium hydroxide.

FIG. 1

European Patent
Office

**EUROPEAN SEARCH REPORT**

Application Number

EP 90 31 1133

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | US-A-3 808 269 (R.W. BRAGDON) <br> * Claims * <br> --- | 1-8 | C 07 C 227/38 <br> C 07 C 229/16 |
| A,D | US-A-4 299 978 (K. NAKAYASU et al.) <br> * Claims * <br> --- | 1-8 | |
| A | DE-A-2 713 472 (W.R. GRACE & CO.) <br> * Claims * <br> ----- | 1-8 | |

TECHNICAL FIELDS
SEARCHED (Int. Cl.5)

C 07 C 227/00

The present search report has been drawn up for all claims

| Place of search | Date of completion of the search | Examiner |
|---|---|---|
| THE HAGUE | 13-02-1991 | SANCHEZ Y GARCIA J.M. |

EPO FORM 1503 03.82 (P0401)